# EUROPEAN PATENT SPECIFICATION

(11) **EP 4 272 780 B1**
(45) Date of publication and mention of the grant of the patent: **09.12.2026**
(21) Application number: 22749647.8
(22) Date of filing: 31.01.2022
(51) Int. Cl.: A61M 5/148, A61M 5/145, A61M 31/00

(54) **LIQUID MEDICINE ADMINISTRATION DEVICE**
VORRICHTUNG ZUR VERABREICHUNG EINES FLÜSSIGEN ARZNEIMITTELS
DISPOSITIF D'ADMINISTRATION DE MÉDICAMENT LIQUIDE

(30) Priority: 02.02.2021 JP 2021014715
(43) Date of publication of application: 08.11.2023
(73) Proprietor: TERUMO Kabushiki Kaisha, Tokyo 151-0072 (JP)
(72) Inventor: SEKIGUCHI, Shota, Ashigarakami-gun, Kanagawa 259-0151 (JP)
(74) Representative: Casalonga
(86) International application number: PCT/JP2022/003537
(87) International publication number: WO 2022/168775

(56) References cited:
- JP-A- 2002 239 003
- JP-A- 2002 239 003
- JP-B1- 6 298 208
- JP-B2- H07 102 417
- US-A1- 2010 121 274
- US-A1- 2015 112 263

## Description

### Technical Field

The present invention relates to a liquid medicine administration device that administers a liquid medicine filled in a cylindrical body into a living body by pressing the liquid medicine. In particular, the present invention relates to a liquid medicine administration device according to the preamble of independent claim 1, such as it is for example known from JP 2002 239003 A, JP 6298208 B1 or US 2010 0121274 A1.

### Background Art

The present applicant has proposed a syringe pump type liquid medicine administration device that administers a liquid medicine filled in a cylindrical body into a living body under a pressing action of a plunger (see WO 2017/170629 A).

The liquid medicine administration device includes a cylindrical body filled with the liquid medicine, a gasket slidably disposed in the cylindrical body, and a plunger mechanism connected to the gasket. The plunger mechanism includes a rotary member rotatable under a driving action of a drive unit, and a moving member movable in an axial direction with respect to the rotary member. Then, the rotary member of the plunger mechanism rotates under the driving action of the drive unit, and accordingly, the gasket is pressed toward the distal end side by advancing the moving member, and the liquid medicine in the cylindrical body is discharged through the discharge opening.

### Summary of Invention

In the above-described liquid medicine administration device, the moving member extends in the axial direction with respect to the cylindrical body, and it is necessary to secure a space for the moving member to move in the axial direction outside the cylindrical body. Therefore, the liquid medicine administration device becomes large in the axial direction. In accordance with the above problem, there is a demand for downsizing the liquid medicine administration device in the axial direction. In addition, when the gasket made of an elastic material is pushed out toward the distal end side in the cylindrical body to discharge the liquid medicine, there is a demand for more smoothly discharging the liquid medicine because sliding resistance is generated between the gasket and the inner peripheral surface of the cylindrical body.

A general object of the present invention is to provide a liquid medicine administration device capable of more smoothly and efficiently administering a liquid medicine while achieving downsizing.

In order to solve this problem, the present invention provides a liquid medicine administration device according to independent claim 1. The dependent claim relates to an advantageous embodiment.
According to the present invention, the liquid medicine administration device includes a tube filled with a liquid medicine and housed in a space of a cylindrical body. The tube is formed in a cylindrical shape having a discharge hole for discharging the liquid medicine at a distal end, and is compressed in an axial direction from a proximal end toward a distal end side along with movement of a pressing member, and is formed to be deformable toward a central axis side.

Therefore, by using a tube that is compressed in the axial direction by the movement of the pressing member and is deformable toward the central axis side, for example, the plunger does not protrude to the outside of the cylindrical body as compared with a general syringe pump type liquid medicine administration device having a plunger such as a moving member of the prior art. Therefore, the liquid medicine administration device can be downsized in the axial direction. In addition, since there is no sliding resistance between the gasket and the cylindrical body generated when the liquid medicine administration is performed by the liquid medicine administration device of the prior art, the liquid medicine in the tube can be smoothly pushed out and administered by compressing the tube in the axial direction and deforming the tube toward the central axis under the movement action of the pressing member. Furthermore, as compared with a case where a bellows-shaped tube is used, the tube can be more greatly compressed and deformed in the axial direction, in a manner that it is possible to efficiently administer the liquid medicine by suitably pushing out the liquid medicine in the tube and reducing the residual amount.

### Brief Description of Drawings

Fig. 1 is an external perspective view of a liquid medicine administration device according to an embodiment of the present invention.
Fig. 2 is an exploded perspective view of the liquid medicine administration device illustrated in Fig. 1.
Fig. 3 is a partially omitted plan view of the liquid medicine administration device illustrated in Fig. 1.
Fig. 4 is a cross-sectional view of the liquid medicine administration device illustrated in Fig. 3.
Fig. 5 is a cross-sectional view taken along line V-V of Fig. 4.
Fig. 6 is an external perspective view of a tube constituting the liquid medicine administration device of Fig. 1.
Fig. 7 is an overall side view of the tube illustrated in Fig. 6.
Fig. 8 is a cross-sectional view illustrating a state in which the tube is compressed toward a distal end side by a plunger mechanism in the liquid medicine administration device illustrated in Fig. 4.
Fig. 9 is a cross-sectional view taken along a line IX-IX in Fig. 8.
Fig. 10 is an overall side view illustrating a state in which the tube illustrated in Fig. 7 is compressed and deformed in the axial direction.

### Description of Embodiments

A liquid medicine administration device 10 is used to administer a liquid medicine into a living body, and administers the liquid medicine filled in a tube 14 in a cylindrical body 12 into the living body over a predetermined time under a pressing action of the plunger mechanism 16. Then, for example, a patch-type needle tube is connected to the liquid medicine administration device 10 as an administration line (not illustrated) at the time of use, and the liquid medicine discharged from the cylindrical body 12 is injected into the body of the patient via the needle tube.

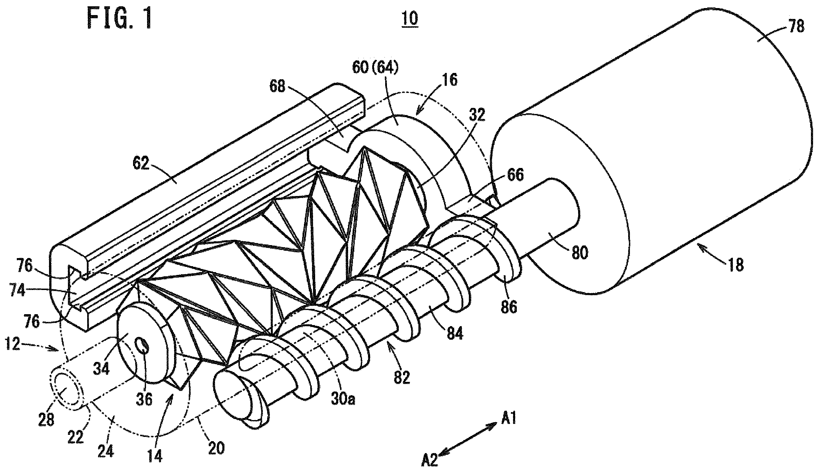

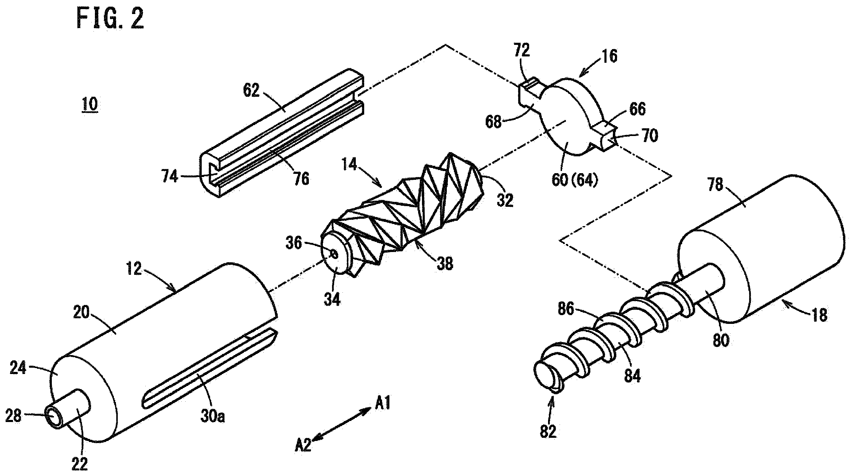

As illustrated in Figs. 1 to 5, the liquid medicine administration device 10 includes the cylindrical body 12, the tube 14 housed in the cylindrical body 12 and filled with a liquid medicine, the plunger mechanism 16 that is provided movably with respect to the cylindrical body 12 and presses the tube 14 in the axial direction, and a drive unit 18 that drives the plunger mechanism 16.

The cylindrical body 12 is formed in a hollow cylindrical shape, and includes a body portion 20 having a constant inner diameter and outer diameter in the axial direction (direction of arrows A1 and A2), and a distal end portion 22 protruding from the distal end of the body portion 20 by a predetermined length in the axial direction (direction of the arrow A2). The proximal end side (direction of the arrow A1) of the body portion 20 is opened, and an end wall 24 orthogonal to the axial direction is provided on the distal end side (direction of the arrow A2). Then, the distal end portion 22 is provided at the center of the end wall 24, and a discharge opening 28 that communicates with a housing space (space) 26 formed inside the body portion 20 and in which the tube 14 is housed is formed inside the distal end portion. The discharge opening 28 penetrates along the axial direction (direction of the arrows A1 and A2) and opens on the distal end side.

The peripheral wall of the body portion 20 includes a pair of insertion grooves 30a and 30b formed from the proximal end toward the distal end side (direction of the arrow A2). The insertion grooves 30a and 30b are disposed to be symmetric in the radial direction with respect to the axial center of the body portion 20, and are formed in a straight line with the same length along the axial direction (direction of the arrows A1 and A2).

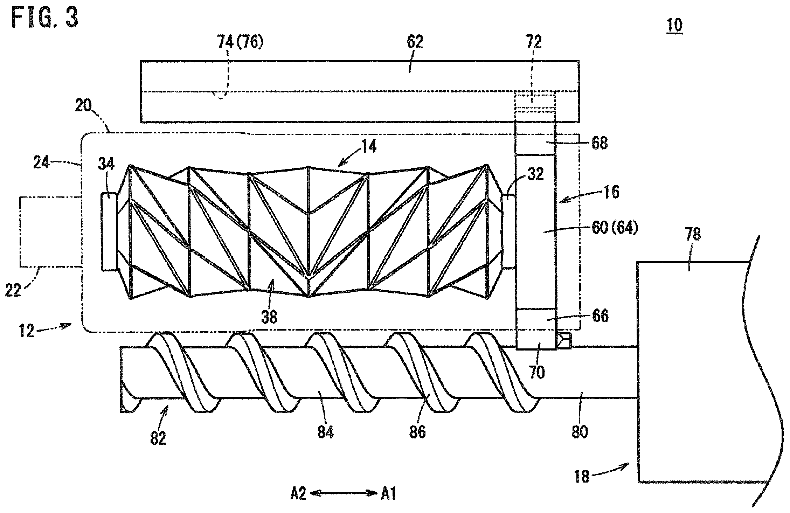

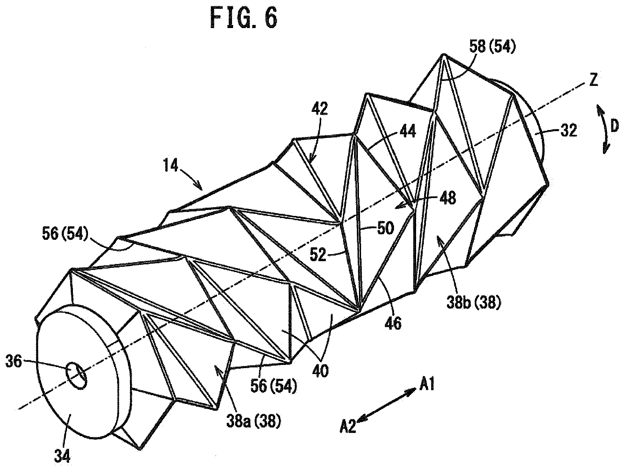

As illustrated in Figs. 1 to 10, the tube 14 is formed in an elastically deformable and stretchable tubular shape from an elastic material such as silicone rubber or fluorine rubber, for example. The tube 14 has a predetermined length in a state of being extended in the axial direction (direction of the arrows A1 and A2) without being applied with an external force illustrated in Figs. 3, 6, and 7. A bottomed cylindrical proximal end side protrusion 32 protruding in the axial direction is formed on the proximal end side (direction of the arrow A1) of the tube 14, and a distal end side protrusion 34 protruding in the axial direction with an elliptical cross section is formed on the distal end side (direction of the arrow A2) of the tube 14.

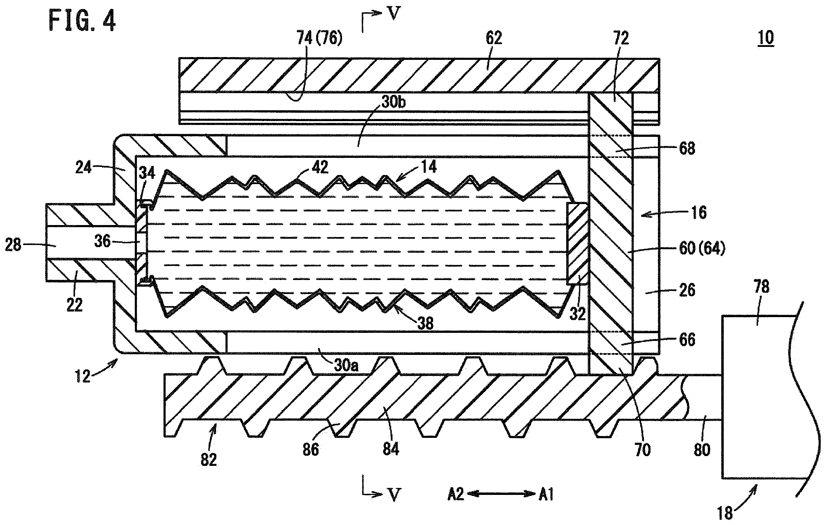

A through hole (discharge hole) 36 penetrating in the axial direction (direction of the arrows A1 and A2) and communicating with the inside is formed at the center of the distal end side protrusion 34. Then, as illustrated in Figs. 3 and 4, the tube 14 is housed in the housing space 26 of the cylindrical body 12 along the axial direction, and is provided in a manner that the proximal end side protrusion 32 abuts on a pressing member 60 of the plunger mechanism 16 described later and the distal end side protrusion 34 abuts on the inner surface of the end wall 24 of the cylindrical body 12. In other words, the tube 14 is held between the pressing member 60 and the end wall 24 inside the cylindrical body 12.

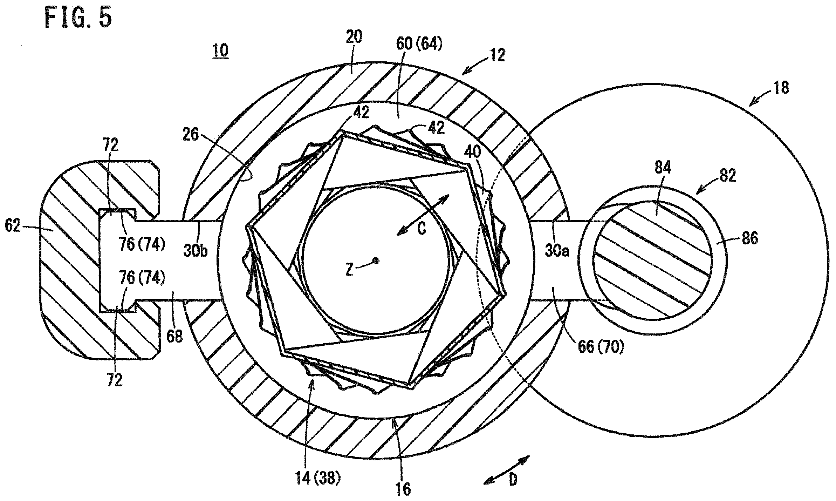

In addition, the tube 14 is formed to be elastically deformable to be compressed in the axial direction and toward the side of a central axis Z (in Fig. 5, the inner side along a radial direction C) by pressing by the pressing member 60 of the plunger mechanism 16 described later. The tube 14 includes a plurality of deformation unit portions 38 that is deformable in the axial direction of the central axis Z (direction of the arrows A1 and A2). The deformation unit portion 38 can be deformed along the axial direction (direction of the arrows A1 and A2) of the central axis Z of the tube 14 by being disposed at a predetermined position, a predetermined arrangement pattern, and a predetermined number on the peripheral wall of the tube 14.

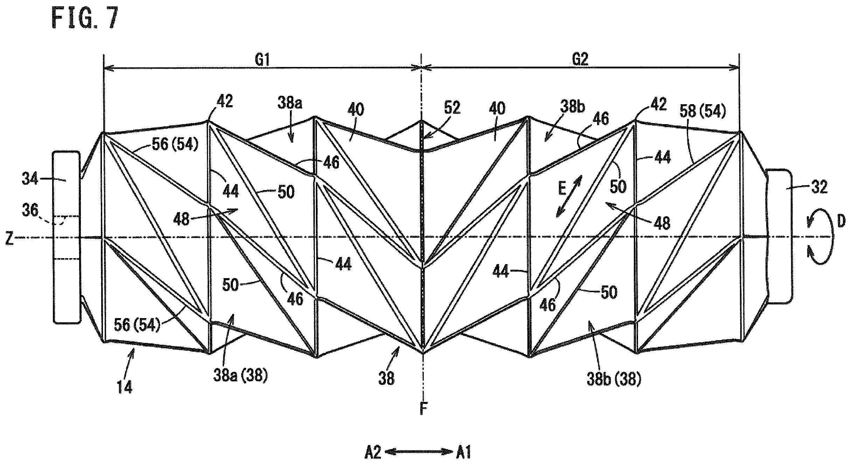

The deformation unit portion 38 is configured in a manner that a ridge portion 42 formed by a ridgeline where adjacent substantially triangular flat surfaces 40 intersect each other is an outer edge. Specifically, in the side view illustrated in Fig. 7, the deformation unit portion 38 includes a set of first ridge portions 44, a set of second ridge portions 46, and a deformation concave portion 48. That is, the ridge portion 42 forming the outer edge of the deformation unit portion 38 includes the set of first ridge portions 44 and the set of second ridge portions 46.

The set of first ridge portions 44 has a predetermined angle with respect to the axial direction (direction of the arrows A1 and A2) of the central axis Z of the tube 14, and one first ridge portion 44 and the other first ridge portion 44 are disposed at a predetermined interval. In addition, it is preferable that the set of first ridge portions 44 extend substantially in parallel in the side view illustrated in Fig. 7. In this way, the tube 14 can be more easily compressed and deformed in the axial direction of the central axis Z (direction of the arrows A1 and A2), and the tube 14 is prevented from being bent and deformed in a direction other than the axial direction of the central axis Z (direction of the arrows A1 and A2).

The set of second ridge portions 46 has a predetermined angle with respect to the axial direction (direction of the arrows A1 and A2) of the axial direction of the central axis Z, and extends to straddle the set of first ridge portions 44. The deformation concave portion 48 is surrounded by the set of first and second ridge portions 44 and 46 and is formed to be deformable in the axial direction of the central axis Z (direction of the arrows A1 and A2).

In addition, the plurality of (six in the present embodiment) deformation unit portions 38 are connected to each other in the extending direction of the first ridge portion 44, that is, over the entire region in a circumferential direction D (see Fig. 5) of the tube 14, and two deformation unit portions 38 adjacent in the circumferential direction D are connected to each other in the circumferential direction D by sharing one second ridge portion 46.

The plurality of deformation unit portions 38 is connected along a spiral direction E that is displaced to one side in the circumferential direction D toward one side in the extending direction of the second ridge portion 46 and the axial direction of the central axis Z (direction of the arrows A1 and A2), and two deformation unit portions 38 adjacent in the spiral direction E are connected in the spiral direction E by sharing one first ridge portion 44.

The deformation concave portion 48 includes a valley portion 50 formed by a ridgeline protruding toward a side opposite to the first ridge portion 44 and the second ridge portion 46 in the radial direction C. The first ridge portion 44 and the second ridge portion 46 are formed by ridgelines protruding outward in the radial direction C of the tube 14. In other words, the valley portion 50 is formed by a ridgeline protruding inward in the radial direction C of the tube 14.

The valley portion 50 is constituted by a diagonal line that forms an angle smaller than that of the first ridge portion 44 and larger than that of the second ridge portion 46 with respect to the axial direction (direction of the arrows A1 and A2) of the central axis Z among diagonal lines connecting intersections of the first ridge portion 44 and the second ridge portion 46 of the deformation unit portion 38.

By providing the above-described valley portion 50, when the tube 14 is compressed and deformed in the axial direction of the central axis Z (direction of the arrows A1 and A2), the deformation concave portion 48 is easily deformed with the valley portion 50 as a fold. As a result, when the tube 14 is compressively deformed in the axial direction (direction of the arrows A1 and A2) of the central axis Z, bending of the deformation concave portion 48 at a position other than the valley portion 50 is suppressed, and generation of a large restoring force (elastic force) in a direction other than the axial direction (direction of the arrows A1 and A2) of the central axis Z can be suppressed.

The deformation unit portion 38 described above includes a first deformation unit portion 38a provided on the distal end side (direction of the arrow A2) with respect to an orthogonal surface (boundary surface) F orthogonal to the axial direction (direction of the arrows A1 and A2) of the central axis Z, and a second deformation unit portion 38b that is plane-symmetric with the first deformation unit portion 38a with respect to the orthogonal surface F and provided on the proximal end side (direction of the arrow A1). The tube 14 includes a first region G1 including only the first deformation unit portion 38a and a second region G2 including only the second deformation unit portion 38b in the axial direction (direction of the arrows A1 and A2) of the central axis Z.

That is, in the tube 14, one side (left side in Fig. 7) is the first region G1 and the other side (right side in Fig. 7) is the second region G2 with the orthogonal surface F as a boundary, which is a central position in the axial direction (direction of the arrows A1 and A2) of the central axis Z. The ranges (axial lengths) of the first region G1 and the second region G2 in the axial direction (direction of the arrows A1 and A2) of the central axis Z are the same.

Since the tube 14 includes the first deformation unit portion 38a and the second deformation unit portion 38b described above, it is possible to suppress twisting deformation around the central axis Z at the time of compressing and deforming the tube 14 in the axial direction of the central axis Z (direction of the arrows A1 and A2) as compared with a configuration including only one of the first and second deformation unit portions 38a and 38b.

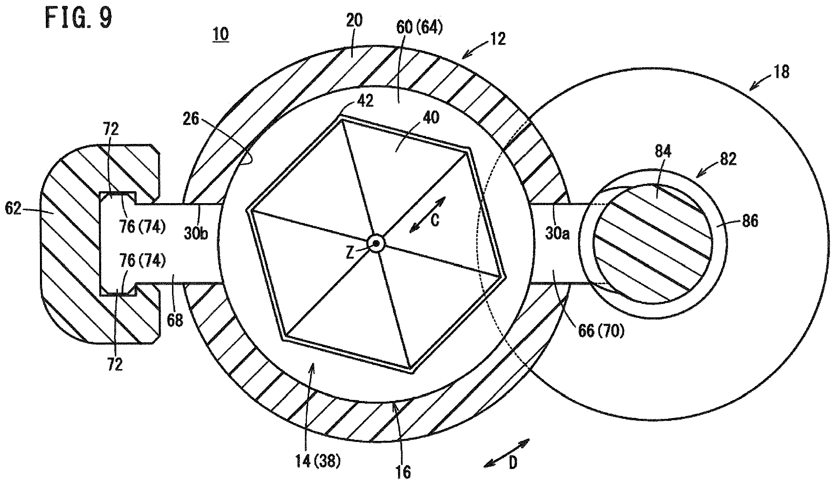

Furthermore, the tube 14 includes an annular convex portion 52 protruding outward in the radial direction C and formed by a ridgeline extending in the circumferential direction D. The annular convex portion 52 is spaced apart from each other at a predetermined interval in the axial direction (direction of the arrows A1 and A2) of the central axis Z, and a plurality of the annular convex portions 52 is provided over the entire region in the axial direction. In addition, the annular convex portion 52 has a substantially hexagonal outer shape in a cross-sectional view of the tube 14 illustrated in Figs. 5 and 9. Note that the outer shape of the annular convex portion 52 is not limited to the above-described hexagonal cross-sectional shape, and may be another polygonal cross-sectional shape.

In addition, among the plurality of annular convex portions 52 described above, the two annular convex portions 52 adjacent in the axial direction (direction of the arrows A1 and A2) of the central axis Z intersect with an inclined convex portion 54 including a ridgeline protruding outward in the radial direction C and extending obliquely with respect to the axial direction (direction of the arrows A1 and A2) of the central axis Z. Specifically, of the plurality of annular convex portions 52, two annular convex portions 52 adjacent in the axial direction of the central axis Z (direction of the arrows A1 and A2) intersect at least two inclined convex portion 54 of the plurality of inclined convex portion 54. The six inclined convex portion 54 intersecting the two annular convex portions 52 adjacent in the axial direction (direction of the arrows A1 and A2) of the central axis Z are disposed at different positions in the circumferential direction D (see Fig. 5).

The inclined convex portion 54 is formed by a ridgeline protruding outward in the radial direction C and extending spirally. The inclined convex portions 54 are spaced apart from each other at a predetermined interval in the circumferential direction D, and are disposed over the entire region in the circumferential direction D. In addition, the inclined convex portion 54 includes a first spiral convex portion 56 extending to be displaced to one side in the circumferential direction D (upper side in Fig. 7) as it goes toward one side in the axial direction of the central axis Z (left side in Fig. 7, and direction of the arrow A2) in the tube 14, and a second spiral convex portion 58 extending to be displaced to one side in the circumferential direction D (upper side in Fig. 7) as it goes toward the other side in the axial direction of the central axis Z (right side in Fig. 7, direction of arrow A1).

The first spiral convex portion 56 is disposed only on the left side with respect to the axial center of the central axis Z of the tube 14, and the second spiral convex portion 58 is disposed only on the right side with respect to the axial center of the central axis Z of the tube 14. In this manner, by providing the first spiral convex portion 56 and the second spiral convex portion 58 having a plane-symmetric shape with the orthogonal surface F orthogonal to the axial direction of the central axis Z as a boundary, it is possible to suppress twisting deformation of the tube 14.

Then, the plurality of annular convex portions 52 and the plurality of inclined convex portions 54 intersect each other, in a manner that a plurality of deformation unit portions 38 having a parallelogram shape are formed on the peripheral wall of the tube 14. The first deformation unit portion 38a formed by the plurality of annular convex portions 52 and the plurality of first spiral convex portion 56 intersecting with each other and the second deformation unit portion 38b formed by the plurality of annular convex portions 52 and the plurality of second spiral convex portion 58 intersecting with each other are formed to be plane-symmetric with respect to the orthogonal surface F orthogonal to the axial direction (direction of the arrows A1 and A2) of the central axis Z.

As illustrated in Figs. 1 to 5, the plunger mechanism 16 includes the pressing member 60 that is provided movably in the axial direction along the cylindrical body 12 and is capable of pressing the proximal end of the tube 14 toward the distal end side (direction of the arrow A2), and a guide member 62 that guides the pressing member 60 movably in the axial direction.

The pressing member 60 is formed of, for example, a plate material having an integral thickness, and includes a pressing portion 64 having a circular cross section and provided inside the cylindrical body 12, a first arm portion 66 protruding from an outer peripheral surface of the pressing portion 64 and engaged with a feed screw shaft 82 of a drive unit 18 to be described later, and a second arm portion 68 protruding in a direction opposite to the first arm portion 66 with respect to the outer peripheral surface of the pressing portion 64 and engaged with the guide member 62. The pressing portion 64 is formed with an outer peripheral diameter corresponding to the inner peripheral diameter of the cylindrical body 12 and is provided movably in the housing space 26.

The first arm portion 66 extends straight outward in the radial direction with respect to the pressing portion 64, and is inserted into one insertion groove 30a of the cylindrical body 12. A projection portion 70 engageable with a screw portion 86 of the feed screw shaft 82 described later is formed at the distal end of the first arm portion 66. The projection portion 70 is, for example, a projecting piece formed in a spiral shape having the same shape as the spiral shape of the screw portion 86.

The second arm portion 68 is formed to be opposite to the first arm portion 66 with the pressing portion 64 interposed between, extends in a straight line radially outward with respect to the pressing portion 64, and is inserted into the other insertion groove 30b of the cylindrical body 12. The distal end of the second arm portion 68 has a pair of engaging convex portions 72 protruding in a direction orthogonal to the extending direction. The distal end of the second arm portion 68 is inserted into and engaged with a guide groove 74 of the guide member 62 described later.

As illustrated in Fig. 5, when viewed from the axial direction of the liquid medicine administration device 10, the guide member 62 is formed in a U-shaped cross section in which the side of the cylindrical body 12 is opened, is formed to be long along the axial direction (direction of the arrows A1 and A2), and is provided in parallel to the side of the cylindrical body 12 while being separated by a predetermined distance. In the guide member 62, the guide groove 74 having a substantially rectangular cross section is formed on the side surface facing the cylindrical body 12. The guide groove 74 has a pair of concave portions 76 each widened in the vertical direction orthogonal to the extending direction with respect to the opening portion on the side of the cylindrical body 12.

Then, in the guide member 62, the second arm portion 68 of the pressing member 60 inserted into the insertion groove 30b of the cylindrical body 12 is inserted into the guide groove 74, and each of the engaging convex portions 72 is engaged with the concave portion 76 of the guide groove 74. As a result, the pressing member 60 is movably guided along the guide member 62 in the axial direction (direction of the arrows A1 and A2) via the second arm portion 68, and the second arm portion 68 is prevented from being detached toward the side of the cylindrical body 12 under the engagement action between the engaging convex portion 72 and the concave portion 76.

The drive unit 18 includes, for example, a motor 78 that is driven and controlled under the control action of a control unit (not illustrated), and the feed screw shaft 82 coupled to a drive shaft 80 of the motor 78. The feed screw shaft 82 is coupled coaxially with the distal end of the drive shaft 80.

The feed screw shaft 82 includes a shaft portion 84 having a constant diameter along the axial direction (direction of the arrows A1 and A2), and the screw portion 86 formed in a spiral shape on the outer peripheral surface of the shaft portion 84. The feed screw shaft 82 is provided parallel to and spaced apart from the cylindrical body 12 by a predetermined distance on the side of the cylindrical body 12 to be opposite to the guide member 62 with the cylindrical body interposed between.

The proximal end of the shaft portion 84 is coupled to the drive shaft 80 of the motor 78, and the shaft portion 84 is formed in a straight line with a predetermined length toward the distal end side (direction of the arrow A2). The projection portion 70 of the first arm portion 66 is engaged with the outer peripheral surface of the shaft portion 84. The screw portion 86 has a form of a male screw protruding radially outward from the outer peripheral surface of the shaft portion 84, and is formed from the vicinity of the proximal end to the distal end of the shaft portion 84.

Then, in the drive unit 18, the feed screw shaft 82 integrally rotates as the drive shaft 80 of the motor 78 rotates based on a control signal from a control unit (not illustrated).

The liquid medicine administration device 10 according to the embodiment of the present invention is basically configured as described above, and operation and effects will be described below.

First, an administration line (not illustrated) is connected to the distal end portion 22 of the cylindrical body 12 in the liquid medicine administration device 10. For example, the liquid medicine administration device 10 is attached to the skin, and the skin is punctured with a needle. In addition, in the initial state before the liquid medicine is administered, as illustrated in Figs. 3 and 4, the pressing member 60 of the plunger mechanism 16 is positioned on the proximal end side (the direction of the arrow A1) of the cylindrical body 12 in the liquid medicine administration device 10. In the initial state, an external force is not yet applied to the tube 14 filled with the liquid medicine, and the tube is in an extended state in which the tube is not compressed in the axial direction (direction of the arrows A1 and A2).

Then, when the liquid medicine administration device 10 receives an operation start command from a control unit (not illustrated), the motor 78 of the drive unit 18 is driven to rotate the drive shaft 80 and the feed screw shaft 82 in a predetermined direction. As the feed screw shaft 82 rotates, the first arm portion 66 advances along the insertion groove 30a under the engagement action between the screw portion 86 and the projection portion 70 of the pressing member 60, and the pressing portion 64 advances toward the distal end side (direction of the arrow A2) in the housing space 26 of the cylindrical body 12. At this time, the distal end of the second arm portion 68 of the pressing member 60 is engaged with the guide groove 74 of the guide member 62. Therefore, the pressing member 60 is guided only in the axial direction (direction of the arrow A2) by the guide member 62, the relative rotation with respect to the cylindrical body 12 is restricted, and the pressing member 60 does not rotate.

As the pressing member 60 advances, the proximal end side protrusion 32 of the tube 14 is pressed toward the distal end side (direction of the arrow A2) by the pressing portion 64 and starts to be compressed.

Specifically, as illustrated in Fig. 7, as the tube 14 is compressed and deformed along the axial direction of the central axis Z, the first ridge portion 44 positioned in the first region G1 approaches toward the axial center side of the central axis Z while rotating to one side in the circumferential direction D (upward in Fig. 7). In other words, when the tube 14 is compressed and deformed in the axial direction (direction of the arrows A1 and A2) of the central axis Z, the annular convex portion 52 of the first region G1 approaches the axial center side of the central axis Z while rotating to one side in the circumferential direction D.

In addition, the first ridge portion 44 positioned in the second region G2 approaches toward the axial center side of the central axis Z while rotating to one side in the circumferential direction D (upward in Fig. 7). In other words, when the tube 14 is compressed and deformed in the axial direction of the central axis Z, the annular convex portion 52 of the second region G2 approaches the axial center side of the central axis Z while rotating to one side in the circumferential direction D.

At this time, the position of the annular convex portion 52 constituting the boundary between the first region G1 and the second region G2 in the axial direction (direction of the arrows A1 and A2) of the central axis Z does not change and rotates to one side in the circumferential direction D (upward in Fig. 7). As described above, the annular convex portion 52 constituting the first ridge portion 44 of the first region G1 and the annular convex portion 52 constituting the first ridge portion 44 of the second region G2 both rotate in the same direction in the circumferential direction D, in a manner that the tube 14 is compressed and deformed in a state where twisting deformation is suppressed.

In addition, the first ridge portion 44 approaches toward the axial center side of the central axis Z as the valley portion 50 of the deformation concave portion 48 is deformed to be bent. As the valley portion 50 is bent, each intersection of the first ridge portion 44 and the second ridge portion 46 approaches another intersection of the first ridge portion 44 and the second ridge portion 46.

Specifically, the tube 14 is suitably compressively deformed in the axial direction of the central axis Z to suppress generation of a large restoring force (elastic force) directed in a direction other than the axial direction in a manner that each intersection of the first ridge portion 44 and the second ridge portion 46 approaches another intersection positioned on the opposite side with the valley portion 50 interposed between.

Then, as the pressing member 60 of the plunger mechanism 16 advances toward the distal end side (direction of the arrow A2), the tube 14 is continuously pressed toward the distal end side, and accordingly, the liquid medicine in the tube 14 is pushed out from the through hole 36 of the distal end side protrusion 34 through the discharge opening 28 of the cylindrical body 12. At this time, for example, as compared with a case where the tube 14 is formed in a bellows shape, the adjacent flat surfaces 40 in each deformation unit portion 38 are deformed to be in close contact with each other by bending the valley portion 50 of the deformation concave portion 48. As a result, the tube 14 can be more greatly compressed and deformed in the axial direction of the central axis Z, in a manner that the residual amount of the liquid medicine in the tube 14 can be reduced. The liquid medicine pushed out of the cylindrical body 12 is administered (injected) into the body of the patient via an administration line (not illustrated) punctured into the patient.

As described above, the present embodiment includes the cylindrical body 12 having the housing space 26 inside, the tube 14 housed in the cylindrical body 12 and filled with the liquid medicine, and the plunger mechanism 16 provided movably in the axial direction with respect to the cylindrical body 12 and having the pressing member 60 that presses the proximal end of the tube 14 toward the distal end side to push out the liquid medicine. Then, by pressing the proximal end of the tube 14 toward the distal end side by the pressing member 60 of the plunger mechanism 16, the tube 14 is compressed toward the distal end side along the axial direction and is deformed toward the central axis Z side of the tube 14, that is, inward along the radial direction C, and the liquid medicine filled in the tube 14 can be pushed out toward the distal end side and pushed out from the discharge opening 28 through the through hole 36.

Therefore, in the liquid medicine administration device 10, by using the tube 14 that is compressed in the axial direction by the movement of the pressing member 60 and is deformable toward the central axis Z side, for example, the pressing member 60 corresponding to the plunger does not protrude to the outside of the liquid medicine administration device 10 as compared with a liquid medicine administration device having a moving member functioning as a plunger as in the prior art. Therefore, the longitudinal dimension along the axial direction of the liquid medicine administration device 10 can be downsized.

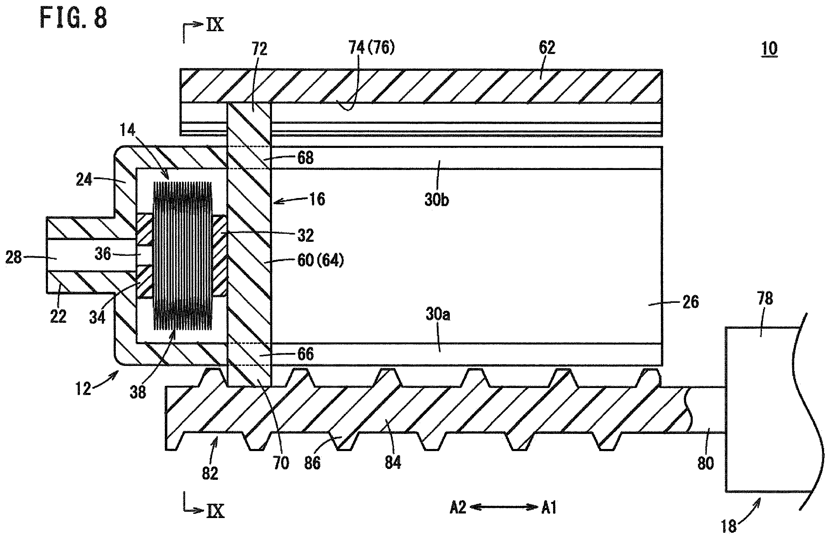

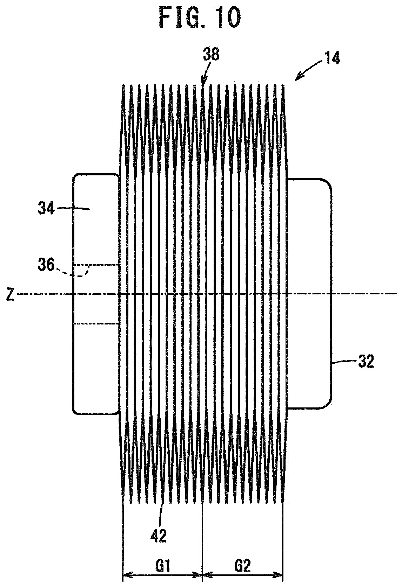

In the liquid medicine administration device 10 described above, there is no sliding resistance generated when the gasket is moved inside the cylindrical body at the time of liquid medicine administration in the conventional liquid medicine administration device. Therefore, the liquid medicine in the tube 14 can be smoothly pushed out and administered under the moving action of the pressing member 60. Furthermore, as compared with a case where a bellows-shaped tube is used, the tube 14 can be more greatly compressed and deformed in the axial direction (see Figs. 8 and 10), in a manner that it is possible to efficiently administer the liquid medicine by suitably pushing out the liquid medicine in the tube 14 and reducing the residual amount.

The tube 14 includes a plurality of deformation unit portions 38 having a plurality of flat surfaces 40 and a plurality of ridge portions 42 formed by ridgelines formed by two adjacent flat surfaces 40 and constituting an outer edge. Therefore, when the tube 14 is compressed and deformed in the axial direction (direction of the arrows A1 and A2) of the central axis Z, the amount of deformation in the axial direction (direction of the arrows A1 and A2) of the central axis Z borne by each deformation unit portion 38 can be reduced.

The deformation unit portion 38 includes the first deformation unit portion 38a provided on one side with respect to the flat surface 40 orthogonal to the axial direction of the central axis Z of the tube 14 and the second deformation unit portion 38b provided on the other side with respect to the flat surface 40, and the tube 14 includes the first region G1 including only the first deformation unit portion 38a and the second region G2 including only the second deformation unit portion 38b in the axial direction of the central axis Z. Therefore, as compared with a case where only one of the first region G1 and the second region G2 is provided, it is possible to suitably suppress twisting deformation when the tube 14 is compressed and deformed in the axial direction of the central axis Z (direction of the arrows A1 and A2).

Note that the liquid medicine administration device according to the present invention is not limited to the above-described embodiment, and it is obvious that various configurations can be adopted without departing from the gist of the present invention.

## Claims

1. A liquid medicine administration device (10) for administering a liquid medicine into a living body, the liquid medicine administration device (10) comprising:
a cylindrical body (12) having a space inside;
a tube (14) housed in a space of the cylindrical body (12) and filled with the liquid medicine; and
a plunger mechanism (16) provided movably in an axial direction with respect to the cylindrical body (12), the plunger mechanism (16) including a pressing member (60) that presses a proximal end of the tube (14) toward a distal end side to push out the liquid medicine, wherein
the tube (14) is formed in a tubular shape having a discharge hole (36) for discharging the liquid medicine at a distal end, and is compressed along an axial direction with movement of the pressing member (60) and is formed to be deformable toward a central axis side,
wherein
the tube (14) includes a plurality of deformation unit portions (38) having a plurality of flat surfaces (40) and a plurality of ridge portions (42) formed by ridgelines formed by two adjacent flat surfaces (40) and constituting an outer edge,
**characterized in that**
each deformation unit portion (38) is configured in a manner that a ridge portion (42) formed by a ridgeline where adjacent substantially triangular flat surfaces (40) intersect each other is an outer edge,
each deformation unit portion (38) includes a set of first ridge portions (44), a set of second ridge portions (46), and a deformation concave portion (48), wherein the ridge portion (42) forming the outer edge of the deformation unit portion (38) includes the set of first ridge portions (44) and the set of second ridge portions (46).

2. The liquid medicine administration device (10) according to claim 1, wherein
the deformation unit portion (38) includes a first deformation unit portion (38a) provided on one side in an axial direction with respect to a boundary surface orthogonal to the axial direction of the central axis (Z) and a second deformation unit portion (38b) provided on the other side in the axial direction with respect to the boundary surface, and
the tube (14) includes a first region (G1) including only the first deformation unit portion (38a) and a second region (G2) including only the second deformation unit portion (38b) in the axial direction of the central axis (Z).

## Patentansprüche

1. Vorrichtung (10) zur Verabreichung eines flüssigen Arzneimittels für das Verabreichen eines flüssigen Arzneimittels in einen lebenden Körper, wobei die Vorrichtung (10) zur Verabreichung eines flüssigen Arzneimittels umfasst:
einen zylindrischen Körper (12), der einen Raum im Inneren aufweist;
einen Schlauch (14), der in einem Raum des zylindrischen Körpers (12) untergebracht und mit dem flüssigen Arzneimittel gefüllt ist; und
einen Kolbenmechanismus (16), der in einer axialen Richtung in Bezug auf den zylindrischen Körper (12) beweglich angeordnet ist, wobei der Kolbenmechanismus (16) ein Druckelement (60) umfasst, das ein proximales Ende des Schlauchs (14) in Richtung einer distalen Endseite drückt, um das flüssige Arzneimittel herauszudrücken, wobei
der Schlauch (14) in einer röhrenförmigen Gestalt ausgebildet ist, die eine Auslassöffnung (36) zum Ausgeben des flüssigen Arzneimittels an einem distalen Ende aufweist, und bei Bewegung des Druckelements (60) entlang einer axialen Richtung zusammengedrückt wird und so ausgebildet ist, dass es sich in Richtung zu der Seite der Mittelachse hin verformbar ist, wobei
der Schlauch (14) eine Vielzahl von Verformungseinheits-Abschnitten (38), die eine Vielzahl von flachen Oberflächen (40) aufweisen, und mehrere Stegabschnitte (42) umfasst, die durch Steglinien gebildet werden, welche von zwei benachbarten flachen Oberflächen (40) gebildet werden und eine Außenkante bilden, **dadurch gekennzeichnet, dass**
jeder Verformungseinheits-Abschnitt (38) in einer Art und Weise ausgebildet ist, dass ein Stegabschnitt (42), der durch eine Steglinie gebildet wird, an der sich benachbarte, im Wesentlichen dreieckige flache Oberflächen (40) schneiden, eine Außenkante ist,
jeder Verformungseinheits-Abschnitt (38) einen Satz erster Stegabschnitte (44), einen Satz zweiter Stegabschnitte (46) und einen konkaven Verformungsabschnitt (48) umfasst, wobei der Stegabschnitt (42), der die Außenkante des Verformungseinheits-Abschnitts (38) bildet, den Satz erster Stegabschnitte (44) und den Satz zweiter Stegabschnitte (46) umfasst.

2. Vorrichtung (10) zur Verabreichung eines flüssigen Arzneimittels nach Anspruch 1, wobei
der Verformungseinheits-Abschnitt (38) einen ersten Verformungseinheits-Abschnitt (38a), der auf einer Seite in einer axialen Richtung in Bezug auf eine Grenzfläche vorgesehen ist, welche orthogonal zu der axialen Richtung der Mittelachse (Z) ist, und einen zweiten Verformungseinheits-Abschnitt (38b) umfasst, der auf der anderen Seite in der axialen Richtung in Bezug auf die Grenzfläche vorgesehen ist, und
der Schlauch (14) einen ersten Bereich (G1), welcher nur den ersten Verformungseinheits-Abschnitt (38a) umfasst, und einen zweiten Bereich (G2), welcher nur den zweiten Verformungseinheits-Abschnitt (38b) umfasst, in der axialen Richtung von der Mittelachse (Z) umfasst.

## Revendications

1. Dispositif d'administration de médicament liquide (10) pour administrer un médicament liquide à un corps vivant, le dispositif d'administration de médicament liquide (10) comprenant :
un corps cylindrique (12) ayant un espace intérieur ;
un tube (14) logé dans un espace du corps cylindrique (12) et rempli du médicament liquide ; et
un mécanisme de piston (16) prévu mobile dans une direction axiale par rapport au corps cylindrique (12), le mécanisme de piston (16) comportant un organe de pression (60) qui presse une extrémité proximale du tube (14) vers un côté d'extrémité distale pour faire sortir le médicament liquide, dans lequel
le tube (14) est formé en une forme tubulaire ayant un orifice d'évacuation (36) pour évacuer le médicament liquide à une extrémité distale, et est comprimé le long d'une direction axiale avec le mouvement de l'organe de pression (60) et est formé pour être déformable vers un côté d'axe central,
dans lequel
le tube (14) comporte une pluralité de parties d'unité de déformation (38) ayant une pluralité de surfaces planes (40) et une pluralité de parties de crête (42) formées par des lignes de crête formées par deux surfaces planes (40) adjacentes et constituant un bord extérieur,
**caractérisé en ce que**
chaque partie d'unité de déformation (38) est configurée de manière à ce qu'une partie de crête (42) formée par une ligne de crête où des surfaces plates (40) sensiblement triangulaires adjacentes se croisent soit un bord extérieur,
chaque partie d'unité de déformation (38) comporte un ensemble de premières parties de crête (44), un ensemble de secondes parties de crête (46), et une partie de déformation concave (48), dans lequel la partie de crête (42) formant le bord extérieur de la partie d'unité de déformation (38) comporte l'ensemble de premières parties de crête (44) et l'ensemble de secondes parties de crête (46).

2. Dispositif d'administration de médicament liquide (10) selon la revendication 1, dans lequel
la partie d'unité de déformation (38) comporte une première partie d'unité de déformation (38a) prévue d'un côté dans une direction axiale par rapport à une surface limite orthogonale à la direction axiale de l'axe central (Z) et une seconde partie d'unité de déformation (38b) prévue de l'autre côté dans la direction axiale par rapport à la surface limite, et
le tube (14) comporte une première région (G1) comportant uniquement la première partie d'unité de déformation (38a) et une seconde région (G2) comportant uniquement la seconde partie d'unité de déformation (38b) dans la direction axiale de l'axe central (Z).
